# EUROPEAN PATENT APPLICATION

(11) **EP 1 803 426 A1**
(43) Date of publication of application: **04.07.2007**
(21) Application number: 06126145.9
(22) Date of filing: 14.12.2006
(51) Int. Cl.: A61F 9/01, A61F 9/008, G05G 1/14

(54) **External device for controlling a laser during laser ablation surgery on the cornea ans associated methods**

(30) Priority: 22.12.2005 US 753010 P
(71) Applicant: Alcon RefractiveHorizons, Inc., Fort Worth, Texas 76134-2099 (US)
(72) Inventor: Liedel, Kevin, Orlando, FL 32820 (US); Pettit, George, Maitland, FL 32751 (US); Luloh, Michael, Chuluota, FL 32766 (US)
(74) Representative: Hanna, Peter William Derek

(57) **Abstract**

A system (10) for controlling a device in ophthalmic refractive surgery includes a processor (17), a controller (19), a treatment laser (11), and a signal receiver (21) is provided. A remote device (22) has a plurality of input elements (23-29). At least one input element when activated is configured to emit a discrete signal that is received by the signal receiver and is mapped to an action to be signaled by the controller. A software package (18) is adapted to translate data from the signal receiver into a signal for directing at least the controller. A method for configuring a system for controlling a device in ophthalmic refractive surgery includes providing a remote device as above. A software package resident on a processor receives and translates signal data from the remote device into control data for a hardware element. The software package is also adapted to output a control signal correlated with the control data to the hardware element. A patient bed (13) may be moved between a first (16) and second (14) laser treatment positions.

## Description

### Cross-Reference to Related Application

This application claims priority to Provisional Patent Application 60/753,010, filed December 22, 2005, entitled "External Device for Controlling a Laser During LaserAblation Surgery on the Cornea and Associated Methods."

### Field of the Invention

The present invention is directed to laser surgery on the eye, and, more particularly, to laser ablation surgery for correcting visual impairment, and, most particularly, to systems and methods for control devices for a laser surgical device.

### Background of the Invention

In refractive surgery on the eye, the laser system is typically controlled with the use of a keyboard and mouse, or hard buttons integrated into the system. Such permanently mounted devices are positionally inflexible and non-configurable, and can be difficult to reach during some parts of the procedure, and especially when switching from one eye to another.

It is known to use remote-control devices for operating electronic devices. External devices are also known to be used in cataract and vitreous procedures.

It would be beneficial to provide a remote-control device for use during a surgical procedure such as refractive surgery on the cornea that confers positional and functional flexibility.

### Summary of the Invention

The present invention is directed to a system and method for controlling a device in ophthalmic refractive surgery. The system comprises a processor, a controller in communication with the processor, a treatment laser in communication with the controller, and a signal receiver in communication with the processor. A remote device is in signal communication with the processor that has a plurality of input elements. Each input element when activated is configured to emit a discrete signal that is receivable by the signal receiver. At least one of the discrete signals is mapped to an action to be signaled by the controller. A software package is resident on the processor and is adapted to translate data from the signal receiver into a signal for directing at least the controller.

A method for configuring a system for controlling a device in ophthalmic refractive surgery comprises the step of providing a remote device that has a plurality of input elements, each input element when activated configured to emit a discrete signal. Each discrete signal is electronically mapped to an action to be implemented by a hardware element in a refractive laser surgery system. A software package resident on a processor is provided that is adapted to receive and translate signal data from the remote device into control data for the hardware element based upon the electronic mapping. The software package is also adapted to output a control signal correlated with the control data to the hardware element.

The benefits of the present invention are numerous. The use of a remote device allows for increased flexibility in driving the system by allowing mobility and control from multiple user-defined positions. For example, controls can be switched to the opposite side for alternate eyes, enabling the use of the appropriate hand, or simply providing the option for control units in multiple locations simultaneously. With regard to current practice in LASIK surgery, the present invention could free a surgical assistant for multi-tasking, such as microkeratome preparation, while still maintaining control of the graphical user interface via the remote device.

In addition to physical flexibility and mobility, external controls also allow for increased configurability, since the user can specify control functionality specific to his/her personal preferences, improving ergonomics and ease of use.

As a result of optimizing control location and configuration, the remote device can be operated without a direct line-of-sight between the operator and the control unit. This can remove potential surgical interruptions to locate fixed hard-mounted controls or separate user interfaces, and further improves the surgical process. In addition, since "hard" components are removed from the system, space is saved, and potential electromagnetic interference issues are removed.

The features that characterize the invention, both as to organization and method of operation, together with further objects and advantages thereof, will be better understood from the following description used in conjunction with the accompanying drawing. It is to be expressly understood that the drawing is for the purpose of illustration and description and is not intended as a definition of the limits of the invention. These and other objects attained, and advantages offered, by the present invention will become more fully apparent as the description that now follows is read in conjunction with the accompanying drawing.

### Brief Description of the Drawing

**FIG. 1** is a schematic of an exemplary system of the present invention.
**FIG. 2** is a side perspective view of an exemplary laser system head having a plurality of mounting locations for a remote control device.
**FIG. 3** is a side perspective view of an exemplary surgeon chair having a mounting location for a remote control device.
**FIG. 4** is a side perspective view of an exemplary display device having a docking station on a back side thereof.
**FIG. 5** is a top/front perspective view of a footswitch embodiment of the invention.

### Detailed Description of the Preferred Embodiments

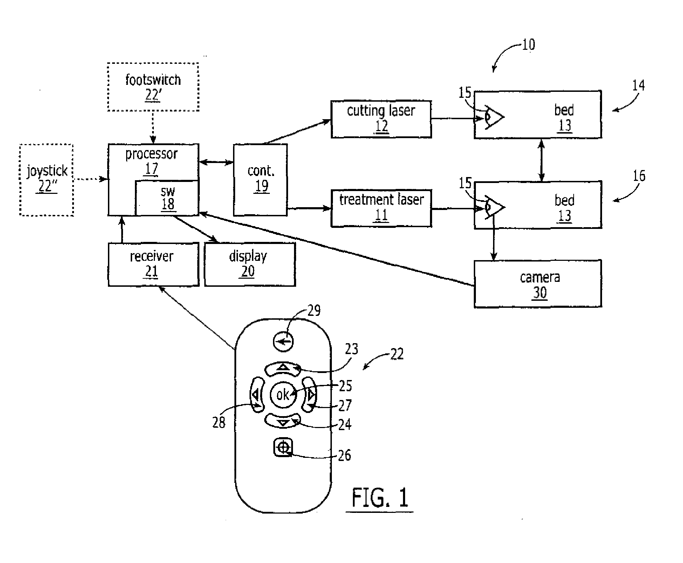

A description of preferred embodiments of the invention will now be presented with reference to FIGS. 1-5. A system **10** for performing a corneal laser ablation comprises a pulsed treatment laser **11** that can be, for example, an excimer laser (FIG. 1). A cutting laser **12** for cutting a corneal flap may also be provided. A patient is typically positioned on a bed **13** that can be movable between a first position **14** for permitting the patient's cornea **15** to be acted upon by the cutting laser **12** and a second position **16** for permitting the patient's cornea **15** to be acted upon by the treatment laser **11.**

The system **10** comprises a processor **17** and software **18** resident thereon. A laser controller **19,** a display device **20,** and a signal receiver **21** are in communication with the processor **17.** A remote control device **22** can be in signal communication with the signal receiver **21** and has a plurality of input elements **23-29.** Each input element **23-29** when activated is configured to emit a discrete signal, for example, in the infrared or radio frequency range, that is receivable by the signal receiver **21.** At least one of the discrete signals is mapped to an action to be signaled by the controller **19.** The software package **18** is adapted to translate data from the signal receiver **21** into a signal for directing hardware elements, such as the controller **19** and the display device **20.**

The display device **20** can comprise a graphical user interface (GUI) for displaying to the surgeon/user, for example, a patient list, a patient bed position, and an image of the patient's cornea **15** as provided by a camera **30.** If the software **18** directs a display of a patient list, up **23** and down **24** arrows on the remote control device **22** can be used to scroll through the patient list, and the "enter" key **25** can be used to select a particular patient name. Upon such a selection, the software **18** retrieves a predetermined ablation profile for the selected patient.

If the user desires to view an image of the eye, a user-defined key **26** on the remote control device **22** can be used to direct the display **20** to show an image from the camera **30,** and the software **18** can process the image to automatically align to a desired location, for example, the cornea.

A direction to move the patient bed **13** between the first **14** and the second **16** position can also be mediated by the remote control device **22** by scrolling through menu items using the up **23,** down **24,** right **27,** and left **28** arrows. A signal to return to a previous GUI menu may be emitted by selecting a back button **29.**

It will be understood by one of skill in the art that, since the input elements **23-29** on the remote control device **22** are not "hard-wired" to control devices, the discrete signals emitted thereby can be user-defined and configurable in any desired manner as mediated by the software package **18.** Further, each the input elements **23-29** can also be programmed to have a multiplicity of functions, for example, under different conditions and for different functionalities, so that, under one set of conditions, the input elements **23-29** are mapped to one set of controls, and, under another set of conditions, the input elements **23-29** can be mapped to a different set of controls as desired. The graphical user interface (GUI) could be programmed, for example, to clearly illustrate the state of the system so that the programmed input element functionalities would be clear to the user.

The remote control device **22** can be constructed in any of a number of ways, and the shape illustrated is not intended to be limiting. Preferably, the device **22** is as thin as possible to avoid blocking direct patient view from the microscope, for example. The buttons **23-29** should preferably be located at the top of the device **22** for ease of access. The buttons **23-29** should be elevated and separate, with unique shapes to permit rapid location. For example, the arrow buttons **23,24,27,28** can have curved inner sides and pointed outer edges. The select button **25** is centered relative to the arrow buttons **23,24,27,28,** and can be the same height as the arrow buttons **23,24,27,28,** with a bump in the middle for ease of location. The auto-align button **26** in the exemplary embodiment is positioned below the arrow buttons **23,24,27,28,** and is shaped similarly to the select **25** without the bump. The back button **29** here is square and is positioned above the arrow buttons **23,24,27,28.** Preferably a depression of the buttons **23-29** will elicit a clicking sound for optimal menu scrolling and user feedback. Also preferably the buttons **23-29** are backlit for ease of viewing in a dim environment.

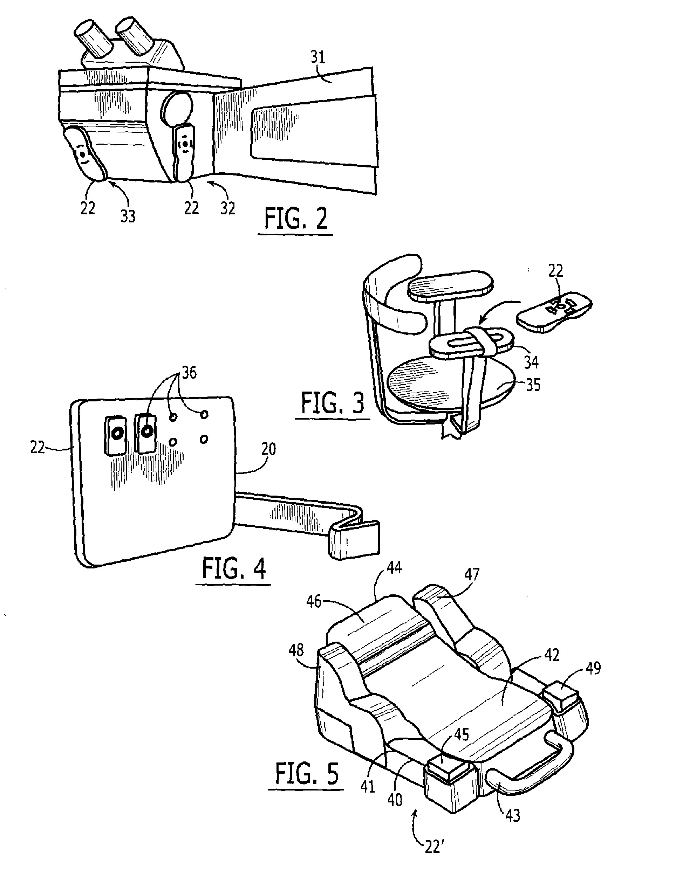

In an alternate embodiment, a foot switch **22'** may be provided in addition to or instead of the hand-held remote control device **22** (FIG. 5). The foot switch **22'** may comprise, for example, a device such as used to control systems in other medical procedures in which the surgeon's hands are occupied, such as cataract surgery. Among the benefits of the foot switch **22'** embodiment are that the control functions can be delegated to an assistant, and also that complete sterility of the surgeon is ensured if the surgeon him/herself is operating the foot switch **22'.** The foot switch **22'** can comprise a base **40** having a substantially planar bottom surface **41** and a top surface **42** that is contoured and dimensioned for permitting a human foot to rest thereupon, and can be angled upward from the front edge **43** toward the rear edge **44.** It is likely that a fixation functionality would not be controlled by a foot switch **22',** although this is not intended as a limitation.

A plurality of input elements **45-49,** here, depressable buttons, are provided that, as above, when activated, are configured to emit a discrete signal, for example, in the infrared or radio frequency range, that is receivable by the signal receiver **21.** At least one of the discrete signals is mapped to an action to be signaled by the controller **19.** The software package **18** is adapted to translate data from the signal receiver **21** into a signal for directing hardware elements, such as the controller **19** and the display device **20.**

In a particular embodiment, as shown in FIG. 5, button **45** is the "back" button; button **46** is the "enter" button; button **47** is the up/down button; button **48** is the left/right button; and button **49** is a user-definable button.

Another option can comprise a joystick **22"** instead of a button-operated remote.

The device **22** itself may be mounted on various locations within the system **10,** for example, as shown in FIGS. 2 and 3, on a microscope arm **31** at any of multiple positions **32,33,** or on an arm **34** of the surgeon's chair **35,** for example, with magnets or another type of interlock. The device **22** can even be mounted to the surgeon's body or clothing or on a mobile cart. Mounting on the laser head allows for a choice of control with either the fingers or thumb of either hand. Further, multiple remote devices **22** can be provided for ease of access at multiple locations. The remote **22** can be recharged at any of a multiplicity of locations on the system **10** as well, as shown in FIG. 4, wherein the back side of the display **20** is shown to have a plurality of docking stations **36** connected to a power source.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the apparatus illustrated and described herein are by way of example, and the scope of the invention is not limited to the exact details of construction.

Having now described the invention, the construction, the operation and use of preferred embodiments thereof, and the advantageous new and useful results obtained thereby, the new and useful constructions, and reasonable mechanical equivalents thereof obvious to those skilled in the art, are set forth in the appended claims.

## Claims

1. A system (10) for controlling a device in ophthalmic refractive surgery comprising:
a processor (17);
a controller (19) in communication with the processor;
a treatment laser (11) in communication with the controller;
a signal receiver (21) in communication with the processor;
a remote device (22) in signal communication with the signal receiver having a plurality of input elements (23-29), each input element when activated configured to emit a discrete signal receivable by the signal receiver, at least one of the discrete signals mapped to an action to be signaled by the controller; and
a software package (18) resident on the processor adapted to translate data from the signal receiver into a signal for directing at least the controller.

2. The system recited in Claim 1, further comprising a display device (20) in communication with the processor (17), at least one of the discrete signals mapped to a control of the display device, and wherein the software package (18) is further adapted to translate data from the at least one of the discrete signals into a control signal for the display device.

3. The system recited in Claim 2, wherein the software package (18) is adapted to direct the display device (20) to display at least one of a patient list, a patient bed position, and an image of a patient cornea.

4. The system recited in claim 2, wherein the software package (18) is adapted to direct the display device (20) to display a patient list, and wherein at least one of the discrete signals is mapped to a selection of a patient, the software package further adapted to receive the patient selection and retrieve a predetermined ablation profile for the selected patient.

5. The system recited in Claim 2, wherein the processor (17) is further in communication with a camera (30) positioned to image a patient eye, and the software package is adapted to automatically calculate and direct a display of an aligned image of a cornea (15) of the patient eye.

6. The system recited in Claim 1, wherein the controller is configured in controlling relation to a patient bed (13) for changing a position thereof.

7. The system recited in Claim 6, wherein the patient bed (13) position is changeable between a treatment position (16) and a corneal flap cutting position (14), the treatment position aligned with a treatment laser (11), the flap cutting position aligned with a tissue cutting laser

8. The system recited in Claim 1, wherein the mapping of at least one of the input elements (23-29) is programmable by a user.

9. The system recited in Claim 1, wherein the remote device (22) is selected from a group consisting of a hand-held remote control device, a foot switch (22'), and a joystick (22").

10. A method for configuring a system for controlling a device in ophthalmic refractive surgery comprising the steps of:
providing a remote device (22) having a plurality of input elements (23-29), each input element when activated configured to emit a discrete signal;
electronically mapping each discrete signal to an action to be implemented by a hardware element in a refractive laser surgery system (10,11,12); and
providing a software package (18) resident on a processor (17), the software package adapted to receive and translate signal data from the remote device into control data for the hardware element based upon the electronic mapping and to output a control signal correlated with the control data to the hardware element.

11. The method recited in Claim 10, wherein at least one of the discrete signals is mapped to a control of a display device (20), and wherein the software package (18) is further adapted to translate data from the at least one of the discrete signals into a control signal for the display

12. The method recited in Claim 11, wherein the software package (18) is adapted to direct the display device (20) to display at least one of a patient list, a patient bed position (14,16), and an image of a patient cornea (15).

13. The method recited in Claim 12, wherein the software package (18) is adapted to direct the display device (20) to display a patient list, and wherein at least one of the discrete signals is mapped to a selection of a patient, the software package further adapted to receive the patient selection and retrieve a predetermined ablation profile for the selected patient.

14. The method recited in Claim 11, wherein the software package (18) is adapted to receive an image of a patient eye from a camera (30) and to automatically calculate and direct a display of an aligned image of a cornea (15) of the patient eye.

15. The method recited in Claim 10, wherein at least one of the discrete signals is mapped to a controller (19) for altering a position (14,16) of a patient bed (13).

16. The method recited in Claim 15, wherein the patient bed position is changeable between a treatment position (16) and a corneal flap cutting position (14), the treatment position aligned with a treatment laser (11), the flap cutting position aligned with a tissue cutting laser (12).

17. The method recited in Claim 10, wherein the software package (18) is further adapted to receive user input in order to configure the mapping of at least one of the input elements (23-29) to a user-specified function.

18. The method recited in Claim 10, wherein the remote device (22) is selected from a group consisting of a hand-held remote control device, a foot switch (22'), and a joystick (22").
